# FASCICULE DE BREVET EUROPEEN

(11) **EP 4 164 716 B1**
(45) Date de publication et mention de la délivrance du brevet: **06.03.2024**
(21) Numéro de dépôt: 21737718.3
(22) Date de dépôt: 08.06.2021
(51) Int. Cl.: A61M 11/00, A61M 15/00

(54) **DISPOSITIF DE DISTRIBUTION DE PRODUIT FLUIDE**
VORRICHTUNG ZUR ABGABE EINES FLÜSSIGEN PRODUKTS
DEVICE FOR DISPENSING A FLUID PRODUCT

(30) Priorité: 10.06.2020 FR 2006060
(43) Date de publication de la demande: 19.04.2023
(73) Titulaire: APTAR France SAS, 27110 Le Neubourg (FR)
(72) Inventeur: FABIEN, David, 29290 Saint Renan (FR)
(74) Mandataire: CAPRI
(86) Numéro de dépôt international: PCT/FR2021/051027
(87) Numéro de publication internationale: WO 2021/250350

(56) Documents cités:
- FR-A1- 3 072 294
- US-A1- 2010 084 433
- US-A1- 2018 060 527

## Description

La présente invention concerne un dispositif de distribution de produit fluide.

De nos jours, l'administration de certains produits fluides, tels que des médicaments puissants potentiellement létaux chez l'homme, peut-être une nécessité dans certaines situations. C'est notamment le cas pour le traitement de pathologies particulières ou encore pour des personnes ayant besoin de traitements palliatifs dans des contextes de fin de vie. La manipulation de telles substances requiert une grande prudence et des dispositifs d'administration extrêmement sûrs, pour éviter les risques de surdose, qui peuvent survenir en cas d'administration rapprochée de plusieurs doses consécutives. Un autre risque concerne l'utilisation de ces dispositifs par une personne autre que la personne à qui le traitement est destiné, par exemple des enfants.

Les documents US 2018060527 et FR 3072294 décrivent des dispositifs de l'état de la technique.

La présente invention a pour but de fournir un dispositif de distribution de produit fluide qui ne reproduit pas les inconvénients susmentionnés.

La présente invention a notamment pour but de fournir un dispositif de distribution de produit fluide qui soit sécurisé et sécurisant pour l'utilisateur, notamment pour éviter les risques d'overdose.

La présente invention a aussi pour but de fournir un dispositif de distribution de produit fluide qui ne soit utilisable que par les personnes autorisées, en particulier par la personne en ayant le besoin thérapeutique.

La présente invention a également pour but de fournir un dispositif de distribution de produit fluide qui soit bloqué pendant un temps prédéterminable entre deux actionnements successifs.

La présente invention a également pour but de fournir un dispositif de distribution de produit fluide qui n'autorise l'actionnement du dispositif que pendant un délai prédéterminable, et qui en l'absence d'actionnement se reverrouille automatiquement à l'expiration dudit délai.

La présente invention a aussi pour but de fournir un dispositif de distribution de produit fluide qui soit robuste et fiable d'utilisation.

La présente invention a également pour but de fournir un dispositif de distribution de produit fluide qui soit simple et peu coûteux à fabriquer et à assembler.

La présente invention a donc pour objet un dispositif de distribution de produit fluide comportant:
- un distributeur de produit fluide comportant un réservoir contenant du produit fluide, une tête de distribution pourvue d'un orifice de distribution, ladite tête de distribution étant axialement déplaçable par rapport audit réservoir, et un organe de distribution, tel qu'une pompe ou une valve, monté sur ledit réservoir, ledit organe de distribution étant actionné lorsque ledit réservoir est déplacé axialement vers le haut par rapport à ladite tête de distribution;
- un corps interne comportant un cylindre creux recevant ledit distributeur et une extension axiale s'étendant axialement vers le bas à partie dudit cylindre creux,
- un module de commande fixé sur ledit corps interne, ledit module de commande comportant une carte électronique, un moteur et une roue de moteur entrainée en rotation par ledit moteur,
- un corps externe recevant ledit corps interne et ledit module de commande,
- un organe de poussée en contact avec ledit réservoir et monté axialement mobile sur ledit corps interne entre une position de repos et une position d'actionnement,
- un organe de commande, fixé audit organe de poussée et se déplaçant axialement ensemble avec lui,
- une bague de verrouillage montée rotative sur ladite extension axiale dudit corps interne entre une position de verrouillage et une position de libération, ladite bague de verrouillage étant déplaçable entre ses positions de verrouillage et de libération par ledit module de commande, ladite bague de verrouillage coopérant en position de verrouillage avec ledit organe de poussée pour empêcher son déplacement axial et coopérant en position de libération avec ledit organe de poussée pour permettre son déplacement axial vers sa position d'actionnement.

Avantageusement, ledit module de commande comporte un élément flexible fixé d'un côté sur ledit corps interne et de l'autre côté sur ladite bague de verrouillage, ladite roue de moteur coopérant avec ledit élément flexible pour le déformer et ainsi déplacer ladite bague de verrouillage entre ses positions de verrouillage et de libération.

Avantageusement, ledit élément flexible est un fil ou une lame flexible.

Avantageusement, ladite extension axiale dudit corps interne comporte un cylindre axial creux pourvu d'au moins une fente axiale s'étendant sur une partie de sa hauteur, ledit cylindre axial creux comportant au moins une paroi radiale s'étendant radialement vers l'extérieur sur toute la hauteur dudit cylindre axial creux, ladite au moins une paroi radiale étant décalée angulairement de chaque fente axiale de 90°.

Avantageusement, ledit cylindre axial creux comporte deux fentes diamétralement opposées et deux parois radiales diamétralement opposées.

Avantageusement, ledit organe de poussée comporte un puits axial s'étendant axialement vers le bas à partir d'un plateau supérieur coopérant avec ledit réservoir, ledit puits axial comportant au moins une extension radiale s'étendant radialement vers l'extérieur à partir de la surface externe dudit puits axial, la surface axiale supérieure de chaque extension radiale étant disposée en éloignement dudit plateau supérieur, chaque extension radiale étant disposée dans une fente axiale respective dudit corps interne.

Avantageusement, ledit organe de poussée comporte deux extensions radiales diamétralement opposées.

Avantageusement, ledit plateau supérieur dudit organe de poussée comporte au niveau d'un bord externe une projection radiale pour fixer un coulisseau coopérant lors de l'actionnement avec ledit module de commande.

Avantageusement, ladite bague de verrouillage comporte un manchon creux pourvu d'au moins une première surface interne plane s'étendant radialement vers l'intérieur à partir d'un bord axial supérieur dudit manchon creux, chaque première surface interne plane s'étendant sur une petite partie de la périphérie interne dudit manchon creux ladite surface axiale supérieure d'une extension radiale dudit organe de poussée étant en butée axiale sur une première surface interne plane respective lorsque la bague de verrouillage est en position de verrouillage.

Avantageusement, en position de libération de ladite bague de verrouillage, ladite au moins une première surface interne plane est décalée angulairement de ladite extension radiale respective, permettant ainsi un déplacement axial dudit organe de poussée par rapport audit corps interne.

Avantageusement, ladite au moins une paroi radiale coopère avec ladite bague de verrouillage pour définir ses positions de verrouillage et de libération.

Avantageusement, ladite bague de verrouillage est bloquée en position de libération pendant tout le cycle d'actionnement, et revient automatiquement dans sa position de verrouillage en fin d'actionnement.

Avantageusement, ledit module de commande déforme ledit élément flexible dès le début du déplacement axial de l'organe de commande pour solliciter ladite bague de verrouillage vers sa position de verrouillage.

Avantageusement, ledit module de commande comporte des moyens de temporisation pour empêcher après chaque actionnement du distributeur le déplacement de ladite bague de verrouillage de sa position de verrouillage vers sa position de libération pendant un temps prédéterminable.

Avantageusement, lesdits moyens de temporisation bloquent un bouton de commande du dispositif et/ou ledit moteur.

Avantageusement, ledit produit fluide est un produit pharmaceutique, tel qu'un médicament.

Ces caractéristiques et avantages et d'autres de la présente invention apparaîtront plus clairement au cours de la description détaillée suivante, faite en référence aux dessins joints, donnés à titre d'exemples non limitatifs, et sur lesquels :

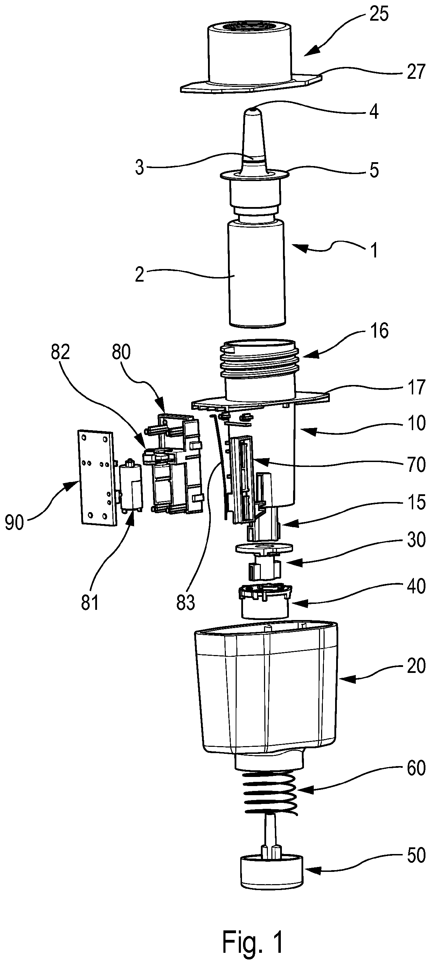

la figure 1 est une vue schématique en perspective éclatée d'un dispositif de distribution selon un mode de réalisation avantageux,
la figure 2 est une vue schématique en perspective de côté du dispositif de la figure 1 après assemblage,
la figure 3 est une vue schématique en perspective éclatée illustrant l'assemblage du distributeur de produit fluide dans le dispositif,
la figure 4 est une vue schématique partielle en perspective éclatée d'une partie interne du dispositif de la figure 1,
la figure 5 est une vue schématique en perspective de dessus de la bague de verrouillage, selon un mode de réalisation avantageux,
la figure 6 est une vue schématique en perspective de dessous de la bague de verrouillage de la figure 5,
la figure 7 est une vue schématique partielle en perspective de côté de l'extension axiale du corps interne, selon un mode de réalisation avantageux,
la figure 8 est une autre vue schématique partielle en perspective de côté de l'extension axiale du corps interne,
la figure 9 est une vue schématique en perspective de côté de l'organe de poussée fixé à l'organe de commande, selon un mode de réalisation avantageux,
la figure 10 est une vue schématique partielle en perspective de dessous de la bague de verrouillage assemblée autour de l'extension axiale du corps interne,
la figure 11 est une vue schématique en section transversale montrant la bague de verrouillage en position de verrouillage,
la figure 12 est une vue schématique en section transversale montrant la bague de verrouillage en position de libération,
la figure 13 est une vue schématique partielle de dessus en perspective découpée de la bague de verrouillage en position de verrouillage,
la figure 14 est une vue schématique partielle de dessus en perspective découpée de la bague de verrouillage en position de libération,
les figures 15 à 17 sont des vues schématiques en perspective découpée qui montrent un cycle d'actionnement du distributeur disposé dans le dispositif, respectivement avant, pendant et après distribution d'une dose de produit fluide,
la figure 18 est une vue schématique partielle de côté en perspective découpée du dispositif avant actionnement, avec la bague de verrouillage en position de verrouillage et les organes de poussée et de commande en position de repos,
la figure 19 est une vue similaire à celle de la figure 18, avec la bague de verrouillage en position de libération,
la figure 20 est une vue similaire à celle de la figure 19, avec les organes de poussée et de commande en position d'actionnement,
la figure 21 est une vue similaire à celle de la figure 20, après actionnement, avec la bague de verrouillage revenue en position de verrouillage et les organes de poussée et de commande revenus en position de repos,
la figure 22 est une vue schématique partielle en perspective montrant la carte électronique, la roue de moteur et le coulisseau,
la figure 23 est une vue schématique partielle de côté en perspective montrant l'élément flexible coopérant avec le corps interne, la roue de moteur et la bague de verrouillage,
la figure 24 est une vue similaire à celle de la figure 23, montrant la bague de verrouillage déplacée vers sa position de verrouillage par l'élément flexible déformé dans un premier sens par la roue de moteur,
la figure 25 est une vue similaire à celle de la figure 24, montrant la bague de verrouillage déplacée vers sa position de libération par l'élément flexible déformé dans le sens opposé par la roue de moteur,
la figure 26 est une vue schématique partielle en perspective montrant la bague de verrouillage en position de verrouillage, telle que sollicitée par l'élément flexible,
la figure 27 est une vue similaire à celle de la figure 26, montrant la bague de verrouillage en position de libération, telle que sollicitée par l'élément flexible,
la figure 28 est une vue similaire à celle de la figure 27, montrant l'élément flexible sollicitant la bague de verrouillage vers sa position de verrouillage, mais avec la bague de verrouillage bloquée en position de libération pendant l'actionnement du dispositif,
la figure 29 est une vue similaire à celle de la figure 20,
la figure 30 est une vue schématique partielle de dessus en perspective découpée de la bague de verrouillage bloquée en position de libération par l'organe de poussée pendant l'actionnement du dispositif,
la figure 31 est une vue schématique partielle de côté en perspective découpée montrant la bague de verrouillage bloquée en position de libération par l'organe de poussée en position d'actionnement,
la figure 32 est une vue similaire à celle de la figure 31, montrant l'organe de poussée revenu en position de repos et ne bloquant donc plus la bague de verrouillage,
la figure 33 est une vue similaire à celle de la figure 32, montrant la bague de verrouillage ramenée en position de verrouillage,
la figure 34 est une vue schématique partielle de côté en perspective montrant le coulisseau fixé à l'organe de poussée, et
les figures 35 et 36 sont des vues schématiques partielles en perspective, montrant le coulisseau respectivement avant actionnement et après le début de son déplacement axial lors de l'actionnement.

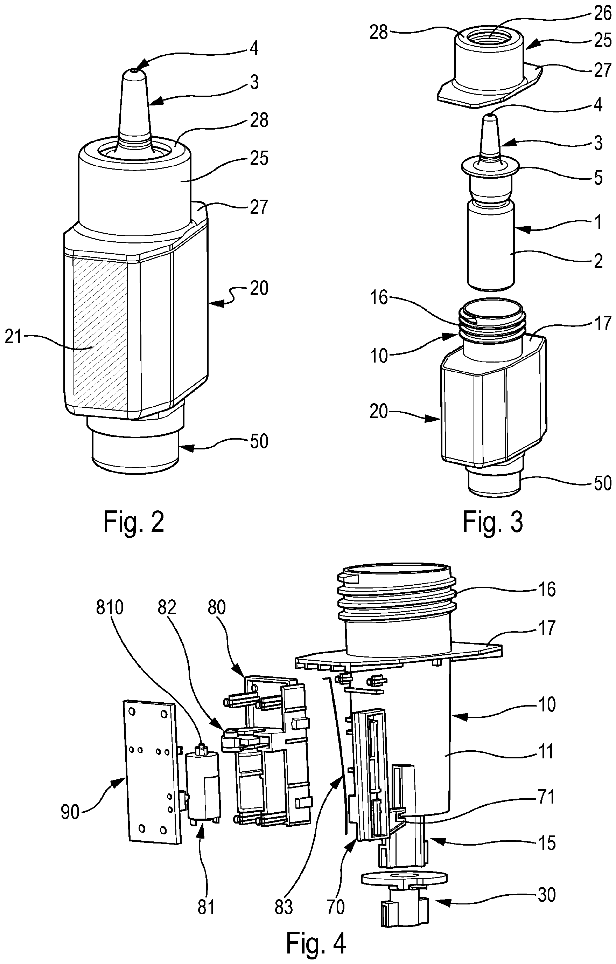

Les termes "axial" et "radial" se réfèrent à l'axe central longitudinal du dispositif. Les termes "haut", "bas", "supérieur" et "inférieur" se réfèrent à la position droite du dispositif représentée sur la figure 2.

La présente invention a principalement pour objet un dispositif qui autorise/interdit la délivrance de dose en bloquant/libérant l'actionnement d'un organe d'actionnement permettant l'actionnement d'un distributeur de produit fluide.

Le dispositif de distribution de produit fluide représenté sur les figures comporte un distributeur de produit fluide 1, avantageusement du type standard. Le produit fluide est de préférence un produit pharmaceutique, tel qu'un médicament. Le distributeur 1 comporte un réservoir 2 contenant le produit fluide et une tête de distribution 3 axialement déplaçable par rapport au réservoir 2. La tête de distribution 3 est pourvue d'un orifice de distribution 4 et d'une surface d'appui radiale 5, qui permet notamment un actionnement manuel dudit distributeur. Un organe de distribution 6, tel qu'une pompe ou une valve, est monté sur le réservoir 2, ledit organe de distribution étant actionné lorsque le réservoir 2 est déplacé axialement vers le haut par rapport à la tête de distribution 3. Généralement, lorsque le réservoir 2 ne contient pas de gaz propulseur, on utilise une pompe doseuse, et lorsque le réservoir 2 contient du gaz propulseur, on utilise une valve doseuse. Ces deux types d'organe de distribution sont bien connus de l'homme du métier et puisque cet organe de distribution n'intervient pas directement dans la présente invention, il ne sera pas décrit plus en détail ci-après.

Le dispositif de distribution de produit fluide comporte également un corps interne 10, recevant le distributeur 1, et un corps externe 20, recevant le corps interne 10.

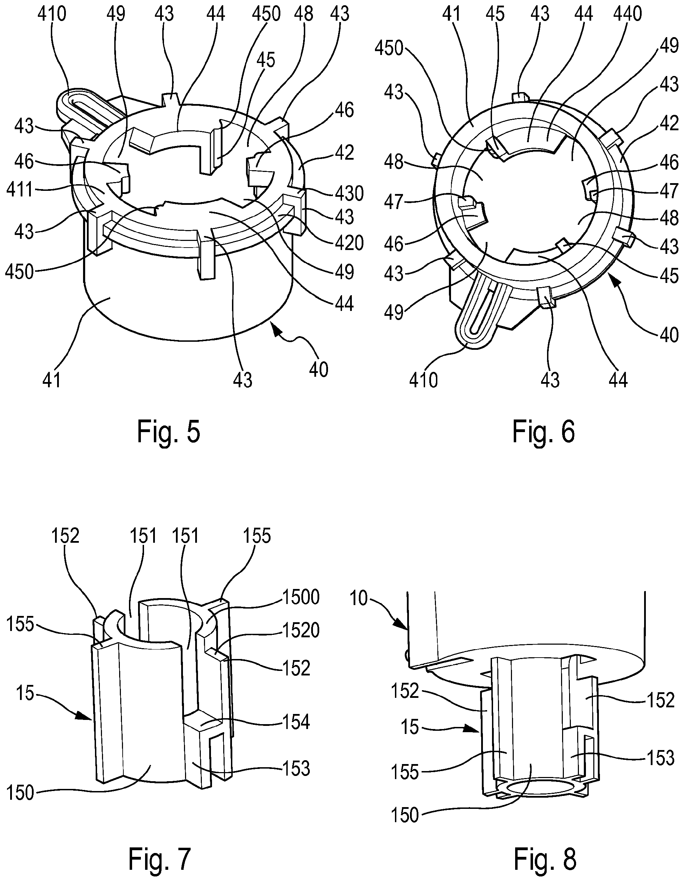

Le corps interne 10 comporte un cylindre creux 11 ouvert axialement du côté supérieur, avec des moyens d'accouplement, avantageusement réalisés sous la forme d'un filetage 16 sur la surface extérieure de l'ouverture supérieur. Une bride radiale 17 s'étend radialement vers l'extérieur en-dessous dudit filetage 16. Du côté inférieur, le corps interne 10 comporte une extension axiale 15, s'étendant axialement vers le bas à partie dudit cylindre creux 11. Cette extension axiale 15, visible sur les figures 7 et 8, est de dimension radiale réduite par rapport au diamètre dudit cylindre creux 11. Elle peut être moulés d'une pièce avec le corps interne 10, ou fabriquée séparément puis fixée audit corps interne 10 d'une quelconque manière appropriée. L'extension axiale 15 comporte un cylindre axial creux 150 pourvu d'au moins une fente axiale 151 s'étendant sur une partie de sa hauteur. Dans l'exemple représenté sur les dessins, il y a deux fentes axiales 151 diamétralement opposées. Chaque fente axiale 151 comporte une première ailette latérale 152 s'étendant vers l'extérieur à partir d'un bord latéral de ladite fente axiale 151. Avantageusement, cette première ailette latérale 152 comporte un bord axial supérieur 1520 décalé axialement vers le bas par rapport au bord axial supérieur 1500 du cylindre axial creux 150. L'avantage de cette mise en oeuvre sera décrite ultérieurement. En-dessous de chaque fente axiale 151, il y a une seconde ailette latérale 153 qui s'étend parallèlement à ladite première ailette latérale 152, lesdites première et seconde ailettes latérales 152, 153 étant reliées par un plat 154 qui forme le fond de chaque fente axiale 151 et le prolonge radialement vers l'extérieur. Le cylindre axial creux 150 comporte au moins une paroi radiale 155 s'étendant radialement vers l'extérieur sur toute la hauteur dudit cylindre axial creux, ladite au moins une paroi radiale 155 étant décalée angulairement de chaque fente axiale de 90°. Dans l'exemple représenté sur les dessins, il y a deux parois radiales 155 diamétralement opposées.

Le corps externe 20 est creux et peut avoir une forme extérieure quelconque. Dans l'exemple représenté, le corps externe 20 est de forme octogonale qui correspond à la forme de ladite bride radiale 17 du corps interne 10. Le corps externe 20 se fixe d'une manière appropriée sur le corps interne 10, par exemple par encliquetage. Avantageusement, le corps externe 20 comporte à l'extérieur un ou plusieurs afficheurs 21, par exemple un écran, permettant d'afficher des informations, comme par exemple des instructions d'utilisation, des informations de charge de batterie, etc. L'afficheur 21 peut aussi intégrer une zone d'actionnement, tel qu'un bouton de commande tactile, sur laquelle l'utilisateur appuie pour déverrouiller le dispositif. En variante, un bouton de commande séparé de l'afficheur 21 pourrait aussi être prévu. Avantageusement, le bouton ou la zone de commande peut intégrer des moyens de détection d'empreinte digitale pour n'autoriser l'actionnement du dispositif qu'à la ou aux personnes autorisées, et empêcher ainsi tout actionnement accidentel, par exemple par des enfants. D'autres moyens de reconnaissance pourraient être envisagés, tels qu'une reconnaissance faciale.

Un capot 25 est prévu pour fixer le distributeur 1 dans l'unité formée du corps interne 1 et du corps externe 20. Ce capot 25 comporte un cylindre creux ouvert axialement des deux côtés, avec des moyens d'accouplement complémentaires, avantageusement réalisés sous la forme d'un filetage interne 26, adaptés à coopérer avec les moyens d'accouplement du corps interne 10. Une première bride radiale 27 est prévue au niveau de l'ouverture inférieure, de forme correspondante à la bride radiale 17 du corps interne 10 et à la forme extérieure du corps externe 20. Une seconde bride radiale 28 est prévue au niveau de l'ouverture supérieure, pour coincer la surface d'appui 5 du distributeur 1 entre le bord axial supérieur du corps interne 10 et ladite seconde bride radiale 28 du capot 25.

Ainsi, pour assembler le distributeur 1 dans le dispositif, on insère le réservoir 2 à l'intérieur du cylindre creux 11 du corps interne 10, jusqu'à ce que la surface d'appui radiale 5 de la tête de distribution 3 vienne reposer sur le bord axial supérieur du corps interne 10. Le capot 25 est alors vissé sur ledit corps interne 10, et en position assemblée du capot 25, la première bride radiale 27 du capot 25 vient recouvrir la bride radiale 17 du corps interne 10 et fermer l'ouverture supérieure du corps externe 20. Cette position assemblée est représentée sur la figure 2.

Le fait de coincer la surface d'appui radiale du distributeur 1 est avantageux en limitant le jeu axial du distributeur dans le dispositif, susceptible de générer des dysfonctionnements.

Le dispositif comporte en outre un organe de poussée 30, une bague de verrouillage 40, un organe d'actionnement 50 et un ressort 60 pour ledit organe d'actionnement.

L'organe de poussée 30 et l'organe d'actionnement 50 sont fixés l'un à l'autre, à la fois axialement et en rotation, et se déplacent ensemble axialement par rapport au corps interne 10 entre une position de repos et une position d'actionnement. Avantageusement, l'organe de poussée 30 est d'abord inséré dans le corps interne 10 par en haut, en étant donc disposé dans ledit corps interne 10 directement sous le réservoir 2. La fixation de l'organe de poussée 30 avec l'organe d'actionnement 50 est dans ce cas réalisé au moment de l'assemblage final du dispositif, après interposition de la bague de verrouillage 40 entre eux, par exemple par un sertissage du type bouterollage à chaud. D'autre fixations sont envisageables.

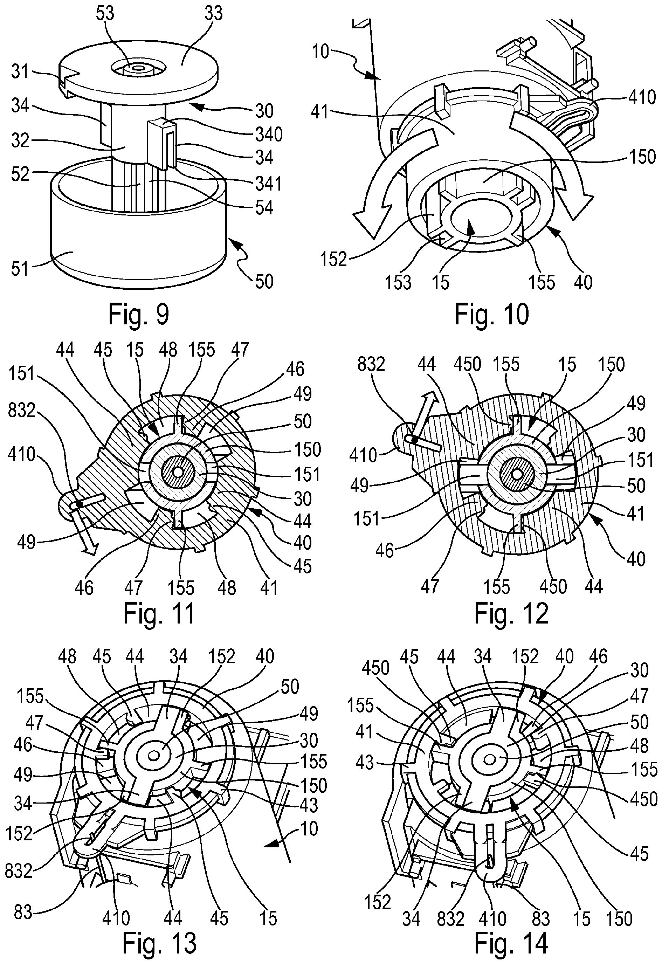

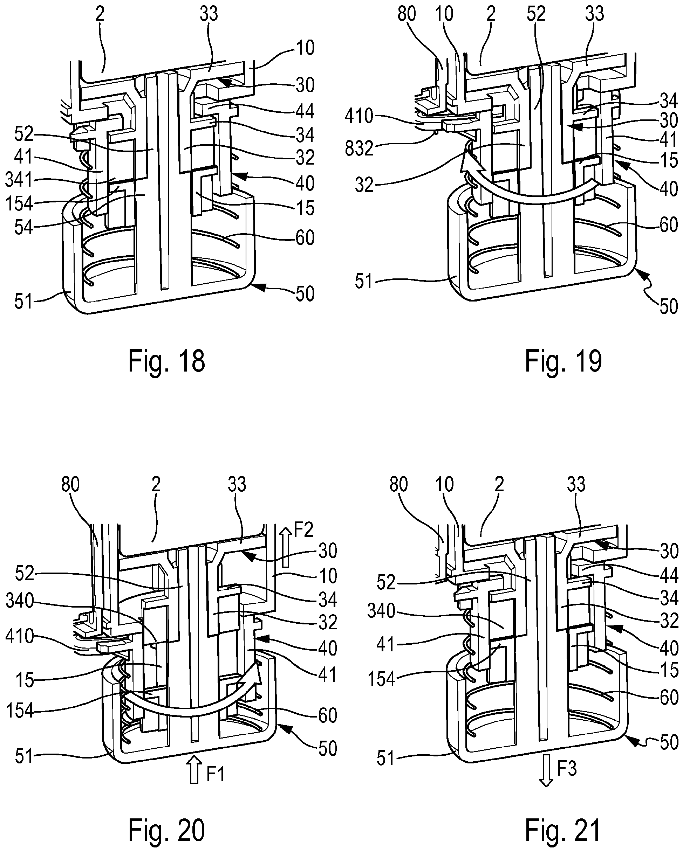

L'organe de poussée 30, visible sur la figure 9, comporte une projection radiale 31 dont la fonction sera explicitée ultérieurement. Il comporte également un puits axial cylindrique 32 s'étendant axialement vers le bas à partir d'un plateau supérieur 33. La projection radiale 31 est avantageusement prévue au niveau d'un bord externe du plateau supérieur 33. Le puits axial 32 comporte au moins une extension radiale 34 s'étendant radialement vers l'extérieur à partir de la surface externe dudit puits axial cylindrique 32. La surface axiale supérieure 340 de chaque extension radiale 34 est disposée en éloignement dudit plateau supérieur 33. Dans l'exemple représenté sur les figures, il y a deux extensions radiales 34 diamétralement opposées. Chaque extension radiale 34 est disposée dans une fente axiale 151 respective du corps interne 10. En position de repos, le bord axial inférieur 341 de chaque extension radiale 34 repose sur un plat 154 respectif du corps interne, bloquant ledit organe de poussée 30 contre tout déplacement axial vers le bas par rapport au corps interne 10, comme visible notamment sur la figure 18. En position d'actionnement, chaque extension radiale 34 s'est déplacée vers le haut dans sa fente axiale 151 respective, comme visible notamment sur la figure 20.

L'organe d'actionnement 50 comporte un cylindre borgne 51 dont le fond forme la surface d'appui pour l'utilisateur. Une tige centrale 52 s'étend axialement vers le haut et est adaptée à traverser le puits axial 32 de l'organe de poussée 30. L'extrémité supérieure 53 de la tige centrale 52 permet de réaliser la fixation de l'organe de poussée 30 sur l'organe d'actionnement 50. Des profils radiaux 54, tels que des nervures, peuvent être prévus sur une partie inférieure de ladite tige axiale 52, pour définir la position de butée de l'organe de poussée 30 par rapport à l'organe d'actionnement 50.

La bague de verrouillage 40 est montée rotative autour de ladite extension axiale 15 du corps interne 10 entre une position de verrouillage et une position de libération. Elle coopère avec ladite extension axiale 15 et avec ledit organe de poussée 30 pour sélectivement bloquer ou permettre le déplacement axial dudit organe de poussée 30 ensemble avec ledit organe d'actionnement 50. La bague de verrouillage 40 comporte un manchon creux 41 pourvu au niveau d'un bord axial supérieur 411 d'une partie de commande 410 s'étendant radialement vers l'extérieur. Comme visible notamment sur les figures 5 et 6, la partie de commande 410 peut former un oeillet, mais d'autres formes sont possibles. A proximité de son bord axial supérieur 411, le manchon creux 41 comporte un anneau radial 42 qui s'étend radialement vers l'extérieur autour dudit manchon creux 41. Le bord axial supérieur 420 dudit anneau radial 42 est décalé axialement vers le bas par rapport au bord axial supérieur 411 du manchon creux 41. Plusieurs projections radiales 43 sont disposés autour dudit anneau radial 42, en s'étendant axialement jusqu'au bord axial supérieur 411 du manchon creux 41 et radialement vers l'extérieur au-delà dudit anneau radial 42. Lesdites projections radiales 43 s'étendent axialement vers le bas au-delà du bord axial inférieur dudit anneau radial 42. Dans l'exemple représenté sur les dessins, il y a six projections radiales 43 réparties sur la périphérie dudit anneau radial 42, avantageusement en étant équidistant angulairement de 60°. La bague de verrouillage 40 comporte au moins une première surface interne plane 44 s'étendant radialement vers l'intérieur à partir du bord axial supérieur 411 du manchon creux 41. Dans l'exemple représenté sur les dessins, il y a deux premières surfaces internes planes 44 diamétralement opposées. Chaque première surface interne plane 44 s'étend sur une petite partie de la périphérie interne dudit manchon creux 41 et comporte sur un côté un premier montant axial 45 s'étendant axialement vers le bas à partir du bord axial supérieur 411 du manchon creux 41, sur une partie de la hauteur dudit manchon creux 41. Ce premier montant axial 45 s'étend aussi radialement vers l'intérieur, comme la surface interne plane 44. Le côté latéral du premier montant axial 45 tourné en éloignement de la surface interne plane 44 comporte avantageusement un profil saillant 450, tel qu'un bourrelet arrondi, visible plus particulièrement sur la figure 5. La surface axiale inférieure 440 de chaque première surface interne plane 44 coopère en position de verrouillage avec une extension radiale 34 de l'organe de poussée 30 pour empêcher un déplacement axial dudit organe de poussée 30. Avantageusement, en position de verrouillage, chaque première surface interne plane 44 s'étend également au-dessus du bord axial supérieur 1520 d'une première ailette latérale 152 respective, ce qui garantit une meilleure résistance à l'effort de l'utilisateur en évitant que la bague de verrouillage 40 ne se retrouve en porte à faux. Il est toutefois à noter que cette mise en oeuvre n'est pas obligatoire. La bague de verrouillage 40 comporte également au moins une seconde surface interne plane 46 s'étendant radialement vers l'intérieur à partir du bord axial supérieur 411 du manchon creux 41. Dans l'exemple représenté sur les dessins, il y a deux secondes surfaces internes planes 44 diamétralement opposées, décalées de 90° par rapport aux premières surfaces internes planes 44. Chaque seconde surface interne plane 46 s'étend sur une petite partie de la périphérie interne dudit manchon creux 41 et comporte sur un côté un second montant axial 47 s'étendant axialement vers le bas à partir à partir du bord axial supérieur 411 du manchon creux 41, sur une partie de la hauteur dudit manchon creux 41. Ce second montant axial 47 s'étend aussi radialement vers l'intérieur, mais moins que la seconde surface interne plane 46, comme visible sur la figure 6. Chaque second montant axial 47 fait face au premier montant axial 45 adjacent, définissant un premier espace angulaire 48 entres eux. De manière similaire, de l'autre côté, il est définit un second espace angulaire 49 entre les première et seconde surfaces internes planes 44, 46. Dans l'exemple représenté, il y a donc deux premiers espaces angulaires 48 diamétralement opposés, et deux seconds espaces angulaires 48 diamétralement opposés. Chaque premier espace angulaire 48 reçoit une paroi radiale 155 de l'extension axiale 15 du corps interne 10. L'amplitude de rotation de la bague de verrouillage 40 est donc défini par ce premier espace angulaire 48. La bague de verrouillage 40 est en position de verrouillage lorsque chaque paroi radiale 155 est en butée contre un second montant axial 47, comme visible sur les figures 11 et 13. Dans cette position de verrouillage, la surface axiale supérieur 340 de chaque extension radiale 34 de l'organe de poussée 30 repose contre la surface axiale inférieure 440 d'une surface interne plane 44 respective, ce qui empêche le déplacement axial vers le haut de l'organe de poussée 30 et donc du réservoir 2. La bague de verrouillage 40 est en position de libération lorsque chaque paroi radiale 155 est en butée contre un premier montant axial 45, comme visible sur les figures 12 et 14. Dans cette position de libération, chaque surface interne plane 44 a tourné par rapport à son extension radiale 34 respective, de sorte que chaque extension radiale 34 se trouve face à un second espace angulaire 49, ce qui permet le déplacement axial vers le haut de l'organe de poussée et donc du réservoir 2. Dès le début du déplacement axial et jusqu'en position d'actionnement de l'organe de poussée 30, chaque extension radiale 34 s'étend dans un second espace angulaire 49 respectif, ce qui bloque en rotation la bague de verrouillage 40 en position de libération, comme visible sur les figures 20, 30 et 31. La bague de verrouillage 40 ne peut ainsi revenir en position de verrouillage que lorsque l'organe de poussée 30 et l'organe de commande 50 sont revenus en position de repos.

Le ressort 60 ramène l'organe d'actionnement 50 et l'organe de poussée 30 en position de repos en fin d'actionnement, lorsque l'utilisateur relâche son effort d'actionnement sur l'organe d'actionnement 50. Il est avantageusement disposé entre le fond du cylindre borgne 51 de l'organe d'actionnement 50 et une partie de la bague de verrouillage 40, avantageusement le bord axial inférieur des projections radiales 43.

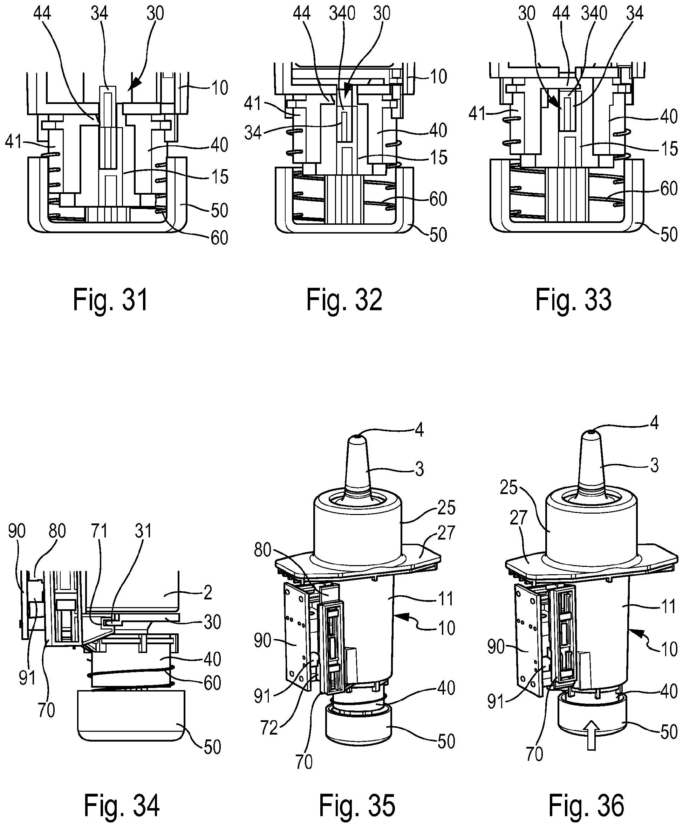

Un coulisseau 70 est monté fixement sur l'organe de poussée 30, et est donc déplaçable axialement ensemble avec lui, en étant disposé à l'extérieur du corps interne 10. Avantageusement, le coulisseau 70 comporte un profil 71, tel qu'un rainure radiale, adaptée à se fixer sur la projection radiale 31 de l'organe de poussée 30, comme visible sur la figure 34. Le coulisseau 70 comporte également une partie de contact saillante 72 dont la fonction sera décrite ci-après.

Un module de commande est fixé sur l'extérieur dudit corps interne 10 et reçu à l'intérieur dudit corps externe 20. Ce module de commande comporte un corps de support 80, fixé au corps interne 10, sur lequel sont assemblés un moteur 81, une roue de moteur 82, un élément flexible 83 et une carte électronique 90.

Le moteur 81 peut être un moteur à engrenage 3V à courant continu adapté à faire tourner un arbre du moteur 810 dans deux directions opposées. Ce moteur peut être alimenté d'une quelconque manière appropriée, par exemple au moyen de batteries ou d'accumulateurs, rechargeables ou non.

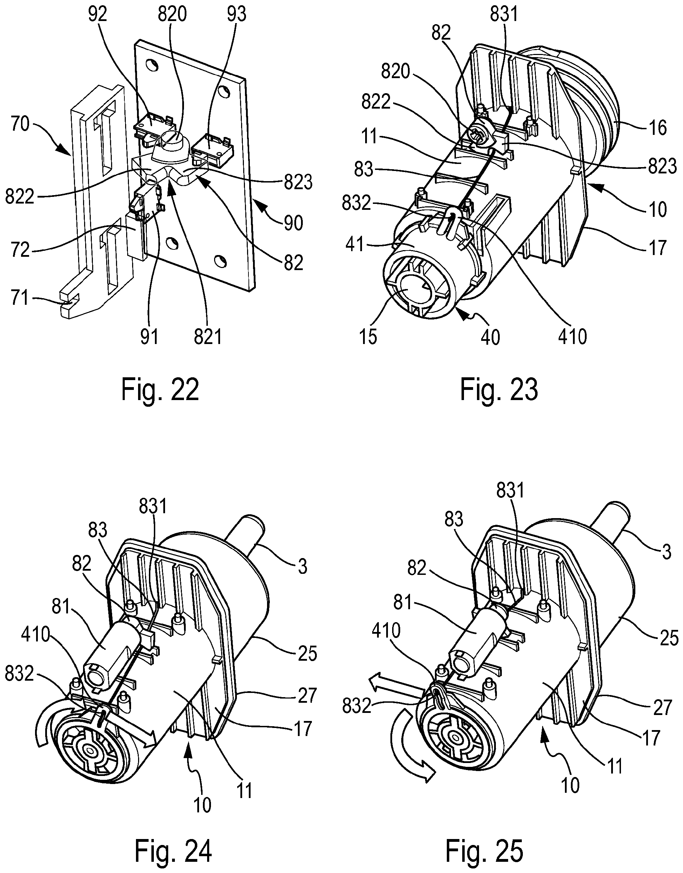

La roue de moteur 82 est montée sur l'arbre de moteur 810, et est donc entrainée en rotation dans l'une ou l'autre direction, selon les instructions transmises au moteur par le module de commande. Cette roue de moteur 82, visible notamment sur les figures 22 et 23, comporte un axe 820 qui reçoit l'arbre de moteur 810, et un évidement 821, défini entre deux dents 822, 823, ledit évidement recevant l'élément flexible 83 pour le déplacer dans un sens ou dans l'autre.

L'élément flexible 83 est avantageusement un fil ou une lame élastiquement déformable, comportant une première extrémité 831 fixée au corps interne 10 et une seconde extrémité 832 fixée à la partie de commande 41 de la bague de verrouillage 40. La première extrémité 831 est donc fixe axialement et en rotation, alors que la seconde extrémité 832 est fixe axialement mais déplaçable en rotation avec la bague de verrouillage 40. L'élément flexible 83 passe dans ledit évidement 821 de la roue de moteur 82, de sorte qu'un déplacement de celle-ci va déformer élastiquement ledit élément flexible dans l'une ou l'autre direction et ainsi exercer une force latérale sur la bague de verrouillage 40 au niveau de ladite partie de commande 410, pour ainsi solliciter ladite bague de verrouillage 40 en rotation dans l'une ou l'autre direction.

La carte électronique 90 comporte des éléments électroniques appropriés, tels que notamment un microprocesseur, pour faire fonctionner le dispositif, notamment le moteur 81 et l'afficheur 21. Avantageusement, la carte électronique 90 comporte aussi un premier switch ou commutateur 91 pour détecter un déplacement du coulisseau 70, et deux seconds switchs ou commutateurs 92, 93 pour détecter la position angulaire de la roue de moteur 82. Le premier switch ou commutateur 91 permet de détecter le début d'actionnement. Ainsi, si après un temps prédéterminable suivant le déverrouillage du dispositif, l'utilisateur n'actionne pas le dispositif, celui-ci peut automatiquement revenir en position de verrouillage. Les premier et seconds switchs ou commutateurs 91, 92, 93 permettent de détecter et d'enregistrer l'actionnement du dispositif, ces informations pouvant être utilisées pour bloquer le dispositif pendant un temps prédéterminé. Ainsi, la carte électronique 90 peut comporter des moyens de temporisation pour n'autoriser un nouvel actionnement qu'après l'expiration d'un délai prédéterminable. Ces moyens de temporisation peuvent notamment comprendre l'horloge interne du microprocesseur. Eventuellement, il est possible de lui adjoindre un composant horloge en temps réel. Ce blocage temporaire agit de préférence sur la commande du moteur 81, empêchant ainsi celui-ci de tourner pour déplacer la bague de verrouillage 40 de sa position de verrouillage vers sa position de libération. En variante, c'est le bouton de commande qui peut être désactivé ou bloqué pendant un temps prédéterminable. Avantageusement, seule des personnes autorisées, tels que le personnel médical, peuvent modifier ledit temps de blocage en ayant accès à la carte électronique ou via l'afficheur 21. De préférence, la carte électronique 90 est disposée à l'intérieur d'une paroi latérale du corps externe 20, et l'afficheur 21 est disposé à l'extérieur de cette même paroi latérale. Avantageusement, l'afficheur 21 indique combien de temps il reste avant de pouvoir et/ou devoir prendre la prochaine dose. Eventuellement, un signal sonore et/ou visuel peut également être prévu si l'utilisateur appuie tout de même sur le bouton de commande pour tenter de déverrouiller le dispositif.

Le fonctionnement du dispositif représenté sur les dessins va maintenant être décrit plus en détail.

Dans un cycle d'actionnement normal, l'utilisateur prend le dispositif dans sa main en position de repos, représentée sur la figure 18. Dans cette position, il ne peut pas déplacer l'organe d'actionnement 50 axialement vers le haut, car celui-ci est bloqué par la bague de verrouillage 40 qui est en position de verrouillage.

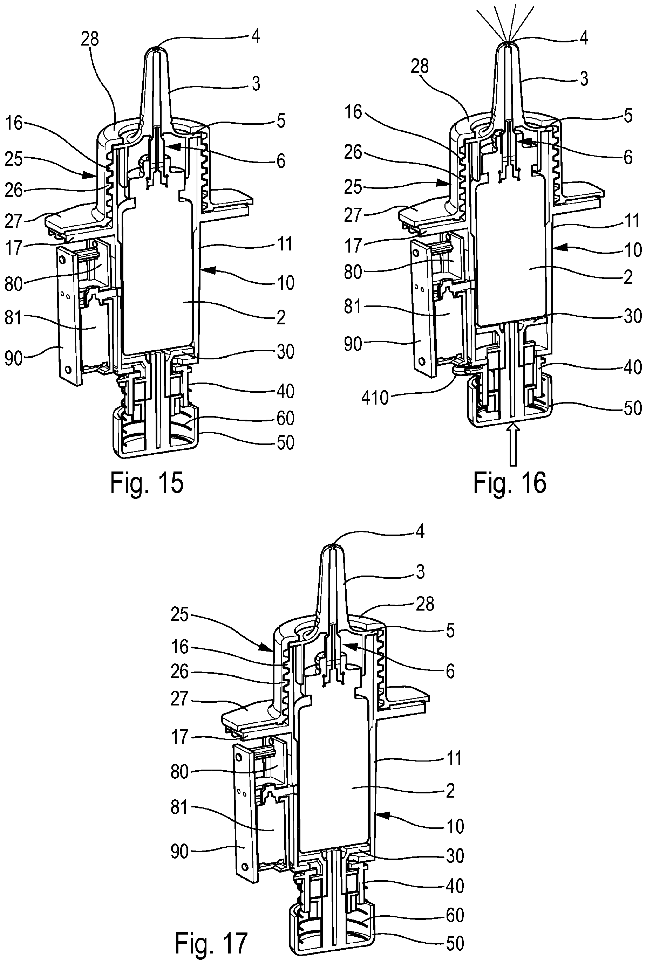

Pour actionner le dispositif, l'utilisateur doit d'abord réaliser une commande du module de commande, pour déplacer la bague de verrouillage 40 de sa position de verrouillage vers sa position de libération. Pour ce faire, il appuie sur un bouton de commande, qui peut avantageusement être intégré dans l'afficheur 21. Cet appui va faire tourner le moteur 81 et donc la roue de moteur 82 dans une direction d'actionnement, ce qui va déformer l'élément flexible 83 dans une première direction, et ainsi solliciter en rotation la bague de verrouillage 40 depuis sa position de verrouillage, visible sur les figures 15, 18 et 26, vers sa position de libération, visible sur les figures 16, 19 et 27.

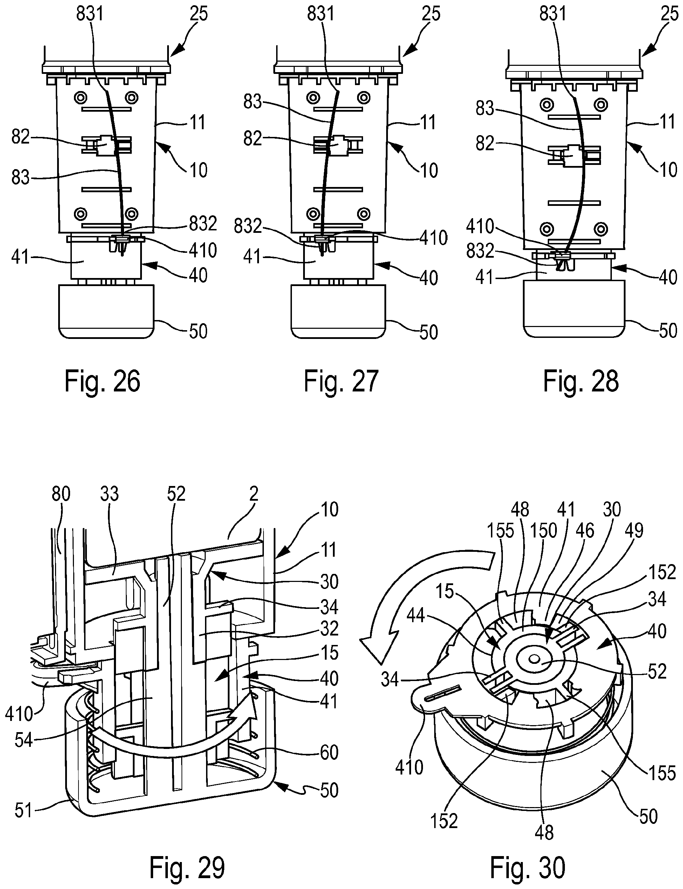

L'utilisateur peut alors exercer un effort d'actionnement axial sur l'organe de commande 50 pour le déplacer axialement vers le haut selon la flèche F1, ce qui va déplacer l'organe de poussée 30 et donc le réservoir 2 de manière concomitante selon la flèche F2, comme visible sur la figure 20. Ceci va actionner l'organe de distribution 6 et expulser une dose de produit fluide à travers l'orifice de distribution 4, comme visible sur la figure 16. Simultanément, le déplacement axial vers le haut de l'organe de poussée 30 entraine le même déplacement axial pour le coulisseau 70 fixé audit organe de poussée 30. En position de repos, la partie de contact saillante 72 du coulisseau ne contacte pas le premier switch ou commutateur 91. Dès que le coulisseau 70 commence à se déplacer axialement vers le haut, la partie de contact saillante 72 entre en contact avec le premier switch ou commutateur 91, comme visible sur la figure 36, ce qui envoie une commande au moteur 81. Celui-ci tourne alors dans la direction opposée à la direction d'actionnement, de même que la roue de moteur 82, sollicitant ainsi l'élément flexible 83 à se déformer dans une seconde direction, opposée à la première direction. L'élément flexible 83 se déforme donc comme visible sur la figure 28, en sollicitant la bague de verrouillage 40 vers sa position de verrouillage. Mais comme la bague de verrouillage 40 est bloquée en position de libération dès le début du déplacement axial de l'organe de poussée 30, elle ne peut pas revenir vers sa position de verrouillage sous l'effet de la force exercée par l'élément flexible 83 déformé. L'élément flexible 83 est ainsi sous contrainte pendant tout le cycle d'actionnement.

Lorsque l'utilisateur relâche son effort d'actionnement axial, le ressort 60 ramène l'organe de poussée 30 et l'organe de commande 50 en position de repos selon la flèche F3, comme visible sur la figure 21. Dans cette position de repos, la bague de verrouillage 40 n'est plus bloquée en rotation par l'organe de poussée 30, comme visible sur la figure 32, et elle revient donc automatiquement vers sa position de verrouillage sous l'effet de la contrainte exercée par l'élément flexible 83 déformé, comme visible sur les figures 17, 21 et 33.

Le dispositif est alors revenu en position de repos, et un prochain actionnement ne sera possible qu'après l'expiration du délai de blocage prédéterminé.

Le dispositif comporte avantageusement divers moyens de sécurité pour permettre et/ou bloquer l'actionnement du dispositif dans certaines conditions particulières.

Ainsi, si l'utilisateur appui fortement sur l'organe d'actionnement 50 avant d'actionner le bouton de commande, il peut potentiellement bloquer la rotation de la bague de verrouillage 40 vers sa position de libération, par frottement de l'extension radiale 34 sur la première surface interne plane 44. Le dispositif est avantageux car lors de l'actionnement du bouton de commande, le moteur 81 déforme l'élément flexible 83, et ainsi la bague de verrouillage 40 tournera vers sa position de libération dès que l'utilisateur relâchera sa pression sur l'organe d'actionnement 50.

Par ailleurs, comme déjà évoqué précédemment, il est possible de reverrouiller le dispositif en ramenant la bague de verrouillage 40 en position de verrouillage si l'utilisateur a laissé le dispositif déverrouillé trop longtemps, par exemple s'il a oublié de prendre la dose.

Bien que la présente invention ait été décrite en référence à un mode de réalisation avantageux, il est entendu que l'homme du métier peut y apporter diverses modifications sans sortir du cadre de la présente invention tel que défini par les revendications annexées.

## Revendications

1. Dispositif de distribution de produit fluide **caractérisé en ce qu'**il comporte :
- un distributeur de produit fluide (1) comportant un réservoir (2) contenant du produit fluide, une tête de distribution (3) pourvue d'un orifice de distribution (4), ladite tête de distribution (3) étant axialement déplaçable par rapport audit réservoir (2), et un organe de distribution (6), tel qu'une pompe ou une valve, monté sur ledit réservoir (2), ledit organe de distribution (6) étant actionné lorsque ledit réservoir (2) est déplacé axialement vers le haut par rapport à ladite tête de distribution (3);
- un corps interne (10) comportant un cylindre creux (11) recevant ledit distributeur (1) et une extension axiale (15) s'étendant axialement vers le bas à partie dudit cylindre creux (11),
- un module de commande (80, 81, 82, 83, 90) fixé sur ledit corps interne (10), ledit module de commande comportant une carte électronique (90), un moteur (81) et une roue de moteur (82) entrainée en rotation par ledit moteur (81),
- un corps externe (20) recevant ledit corps interne (10) et ledit module de commande,
- un organe de poussée (30) en contact avec ledit réservoir (2) et monté axialement mobile sur ledit corps interne (10) entre une position de repos et une position d'actionnement,
- un organe de commande (50), fixé audit organe de poussée (30) et se déplaçant axialement ensemble avec lui,
- une bague de verrouillage (40) montée rotative sur ladite extension axiale (15) dudit corps interne (10) entre une position de verrouillage et une position de libération, ladite bague de verrouillage (40) étant déplaçable entre ses positions de verrouillage et de libération par ledit module de commande, ladite bague de verrouillage (40) coopérant en position de verrouillage avec ledit organe de poussée (30) pour empêcher son déplacement axial et coopérant en position de libération avec ledit organe de poussée (30) pour permettre son déplacement axial vers sa position d'actionnement.

2. Dispositif selon la revendication 1, dans lequel ledit module de commande comporte un élément flexible (83) fixé d'un côté sur ledit corps interne (10) et de l'autre côté sur ladite bague de verrouillage (40), ladite roue de moteur (82) coopérant avec ledit élément flexible (83) pour le déformer et ainsi déplacer ladite bague de verrouillage (40) entre ses positions de verrouillage et de libération.

3. Dispositif selon la revendication 2, dans lequel ledit élément flexible (83) est un fil ou une lame flexible.

4. Dispositif selon l'une quelconque des revendications précédentes, dans lequel ladite extension axiale (15) dudit corps interne (10) comporte un cylindre axial creux (150) pourvu d'au moins une fente axiale (151) s'étendant sur une partie de sa hauteur, ledit cylindre axial creux (150) comportant au moins une paroi radiale (155) s'étendant radialement vers l'extérieur sur toute la hauteur dudit cylindre axial creux (150), ladite au moins une paroi radiale (155) étant décalée angulairement de chaque fente axiale de 90°.

5. Dispositif selon la revendication 4, dans lequel ledit cylindre axial creux (150) comporte deux fentes diamétralement opposées et deux parois radiales (155) diamétralement opposées.

6. Dispositif selon la revendication 4 ou 5, dans lequel ledit organe de poussée (30) comporte un puits axial (32) s'étendant axialement vers le bas à partir d'un plateau supérieur (33) coopérant avec ledit réservoir (2), ledit puits axial (32) comportant au moins une extension radiale (34) s'étendant radialement vers l'extérieur à partir de la surface externe dudit puits axial (32), la surface axiale supérieure (340) de chaque extension radiale (34) étant disposée en éloignement dudit plateau supérieur (33), chaque extension radiale (34) étant disposée dans une fente axiale (151) respective dudit corps interne (10).

7. Dispositif selon la revendication 6, dans lequel ledit organe de poussée (30) comporte deux extensions radiales (34) diamétralement opposées.

8. Dispositif selon la revendication 6 ou 7, dans lequel ledit plateau supérieur (33) dudit organe de poussée (30) comporte au niveau d'un bord externe une projection radiale (31) pour fixer un coulisseau (70) coopérant lors de l'actionnement avec ledit module de commande.

9. Dispositif selon l'une quelconque des revendications 6 à 8, dans lequel ladite bague de verrouillage (40) comporte un manchon creux (41) pourvu d'au moins une première surface interne plane (44) s'étendant radialement vers l'intérieur à partir d'un bord axial supérieur (411) dudit manchon creux (41), chaque première surface interne plane (44) s'étendant sur une petite partie de la périphérie interne dudit manchon creux (41) ladite surface axiale supérieure (340) d'une extension radiale (34) dudit organe de poussée (30) étant en butée axiale sur une première surface interne plane (44) respective lorsque la bague de verrouillage (40) est en position de verrouillage.

10. Dispositif selon la revendication 9, dans lequel en position de libération de ladite bague de verrouillage (40), ladite au moins une première surface interne plane (44) est décalée angulairement de ladite extension radiale (34) respective, permettant ainsi un déplacement axial dudit organe de poussée (30) par rapport audit corps interne (10).

11. Dispositif selon l'une quelconque des revendications 6 à 10, dans lequel ladite au moins une paroi radiale (155) est configurée pour coopérer avec ladite bague de verrouillage (40) pour définir ses positions de verrouillage et de libération.

12. Dispositif selon l'une quelconque des revendications précédentes, dans lequel ladite bague de verrouillage (40) est configurée pour être bloquée en position de libération pendant tout le cycle d'actionnement, et revenir automatiquement dans sa position de verrouillage en fin d'actionnement.

13. Dispositif selon les revendications 2 et 12, dans lequel ledit module de commande est configuré pour déformer ledit élément flexible (83) dès le début du déplacement axial de l'organe de commande (50) pour solliciter ladite bague de verrouillage (40) vers sa position de verrouillage.

14. Dispositif selon l'une quelconque des revendications précédentes, dans lequel ledit module de commande comporte des moyens de temporisation pour empêcher après chaque actionnement du distributeur (1) le déplacement de ladite bague de verrouillage (40) de sa position de verrouillage vers sa position de libération pendant un temps prédéterminable.

15. Dispositif selon la revendication 14, dans lequel lesdits moyens de temporisation sont configurés pour bloquer un bouton de commande du dispositif et/ou ledit moteur (81).

16. Dispositif selon l'une quelconque des revendications précédentes, dans lequel ledit produit fluide est un produit pharmaceutique, tel qu'un médicament.

## Patentansprüche

1. Vorrichtung zur Abgabe eines Fluidprodukts, **dadurch gekennzeichnet, dass** sie Folgendes aufweist:
- einen Fluidproduktspender (1) mit einem Vorratsbehälter (2), der Fluidprodukt enthält, einem Abgabekopf (3), der mit einer Abgabeöffnung (4) versehen ist, wobei der Abgabekopf (3) im Verhältnis zum Vorratsbehälter (2) axial bewegbar ist, und einem Abgabeorgan (6) wie etwa einer Pumpe oder einem Ventil, das auf dem Vorratsbehälter (2) angebracht ist, wobei das Abgabeorgan (6) betätigt wird, wenn der Vorratsbehälter (2) im Verhältnis zum Abgabekopf (3) axial nach oben bewegt wird;
- einen Innenkörper (10) mit einem Hohlzylinder (11), der den Spender (1) und eine axiale Erweiterung (15), die sich von dem Hohlzylinder (11) axial nach unten erstreckt, aufnimmt,
- ein Steuermodul (80, 81, 82, 83, 90), das auf dem Innenkörper (10) befestigt ist, wobei das Steuermodul eine Platine (90), einen Motor (81) und ein Motorlaufrad (82), das vom Motor (81) gedreht wird, aufweist,
- einen Außenkörper (20), der den Innenkörper (10) und das Steuermodul aufnimmt,
- ein Schuborgan (30), das mit dem Vorratsbehälter (2) in Kontakt steht und auf dem Innenkörper (10) axial beweglich zwischen einer Ruheposition und einer Betätigungsposition angebracht ist,
- ein Bedienorgan (50), das am Schuborgan (30) befestigt ist und sich zusammen mit ihm axial bewegt,
- einen Verriegelungsring (40), der auf der axialen Erweiterung (15) des Innenkörpers (10) zwischen einer Verriegelungsposition und einer Freigabeposition drehbar angebracht ist, wobei der Verriegelungsring (40) durch das Steuermodul zwischen seiner Verriegelungs- und Freigabeposition bewegbar ist, wobei der Verriegelungsring (40) in der Verriegelungsposition mit dem Schuborgan (30) zusammenwirkt, um dessen axiale Bewegung zu verhindern, und in der Freigabeposition mit dem Schuborgan (30) zusammenwirkt, um dessen axiale Bewegung zu dessen Betätigungsposition zu ermöglichen.

2. Vorrichtung nach Anspruch 1, wobei das Steuermodul ein biegsames Element (83) aufweist, das auf einer Seite an dem Innenkörper (10) und auf der anderen Seite an dem Verriegelungsring (40) befestigt ist, wobei das Motorlaufrad (82) mit dem flexiblen Element (83) zusammenwirkt, um es zu verformen und so den Verriegelungsring (40) zwischen dessen Verriegelungsund Freigabeposition zu bewegen.

3. Vorrichtung nach Anspruch 2, wobei das flexible Element (83) ein biegsamer Draht oder ein biegsames Blatt ist.

4. Vorrichtung nach einem der vorhergehenden Ansprüche, wobei die axiale Erweiterung (15) des Innenkörpers (10) einen hohlen Axialzylinder (150) aufweist, der mit wenigstens einem axialen Schlitz (151) versehen ist, der sich auf einem Abschnitt seiner Höhe erstreckt, wobei der hohle Axialzylinder (150) wenigstens eine radiale Wand (155) aufweist, die sich über die gesamte Höhe des hohlen Axialzylinders (150) radial nach außen erstreckt, wobei die wenigstens eine radiale Wand (155) von jedem axialen Schlitz um 90° winkelversetzt ist.

5. Vorrichtung nach Anspruch 4, wobei der hohle Axialzylinder (150) zwei diametral gegenüberliegende Schlitze und zwei diametral gegenüberliegende radiale Wände (155) aufweist.

6. Vorrichtung nach Anspruch 4 oder 5, wobei das Schuborgan (30) einen axialen Schacht (32) aufweist, der sich von einer oberen Platte (33), die mit dem Vorratsbehälter (2) zusammenwirkt, axial nach unten erstreckt, wobei der axiale Schacht (32) wenigstens eine radiale Erweiterung (34) aufweist, die sich von der Außenfläche des axialen Schachts (32) radial nach außen erstreckt, wobei die obere axiale Fläche (340) jeder radialen Erweiterung (34) von der oberen Platte (33) entfernt angeordnet ist, wobei jede radiale Erweiterung (34) in einem jeweiligen axialen Schlitz (151) des Innenkörpers (10) angeordnet ist.

7. Vorrichtung nach Anspruch 6, wobei das Schuborgan (30) zwei diametral gegenüberliegende radiale Erweiterungen (34) aufweist.

8. Vorrichtung nach Anspruch 6 oder 7, wobei die obere Platte (33) des Schuborgans (30) an einem Außenrand einen Radialvorsprung (31) zur Befestigung eines Schiebers (70) aufweist, der bei der Betätigung mit dem Steuermodul zusammenwirkt.

9. Vorrichtung nach einem der Ansprüche 6 bis 8, wobei der Verriegelungsring (40) eine hohle Hülse (41) aufweist, die mit wenigstens einer ersten ebenen Innenfläche (44) versehen ist, die sich von einem oberen axialen Rand (411) der hohlen Hülse (41) radial nach innen erstreckt, wobei sich jede ebene Innenfläche (44) über einen kleinen Abschnitt des Innenumfangs der hohlen Hülse (41) erstreckt, wobei die obere axiale Fläche (340) einer radialen Erweiterung (34) des Schuborgans (30) axial an einer jeweiligen ersten ebenen Innenfläche (44) axial anliegt, wenn sich der Verriegelungsring (40) in der Verriegelungsposition befindet.

10. Vorrichtung nach Anspruch 9, wobei in der Freigabeposition des Verriegelungsrings (40) die wenigstens eine erste ebene Innenfläche (44) von der jeweiligen radialen Erweiterung (34) winkelversetzt ist, wodurch eine axiale Bewegung des Schuborgans (30) im Verhältnis zum Innenkörper (10) ermöglicht wird.

11. Vorrichtung nach einem der Ansprüche 6 bis 10, wobei die wenigstens eine radiale Wand (155) dazu ausgebildet ist, mit dem Verriegelungsring (40) zusammenzuwirken, um dessen Verriegelungs- und Freigabeposition zu definieren.

12. Vorrichtung nach einem der vorhergehenden Ansprüche, wobei der Verriegelungsring (40) dazu ausgebildet ist, während des gesamten Betätigungszyklus in der Freigabeposition blockiert zu sein und am Betätigungsende automatisch in seine Verriegelungsposition zurückzukehren.

13. Vorrichtung nach den Ansprüchen 2 und 12, wobei das Steuermodul dazu ausgebildet ist, das biegsame Element (83) ab Beginn der axialen Bewegung des Bedienorgans (50) zu verformen, um den Verriegelungsring (40) zu seiner Verriegelungsposition zu drängen.

14. Vorrichtung nach einem der vorhergehenden Ansprüche, wobei das Steuermodul Verzögerungsmittel aufweist, um nach jeder Betätigung des Spenders (1) eine vorbestimmbare Zeit lang die Bewegung des Verriegelungsrings (40) von seiner Verriegelungsposition zu seiner Freigabeposition zu verhindern.

15. Vorrichtung nach Anspruch 14, wobei die Verzögerungsmittel dazu ausgebildet sind, einen Bedienknopf der Vorrichtung und/oder den Motor (81) zu blockieren.

16. Vorrichtung nach einem der vorhergehenden Ansprüche, wobei das Fluidprodukt ein pharmazeutisches Produkt wie etwa ein Medikament ist.

## Claims

1. A device for dispensing a fluid product **characterised in that** it comprises:
- a fluid product dispenser (1) having a tank (2) containing a fluid product, a dispensing head (3) having a dispensing port (4), said dispensing head (3) being axially movable with respect to said tank (2), and a dispensing member (6), such as a pump or a valve, mounted on said tank (2), said dispensing member (6) being actuated when said tank (2) is moved axially upwards with respect to said dispensing head (3);
- an inner body (10) comprising a hollow cylinder (11) receiving said dispenser (1) and an axial extension (15) extending axially downwards from said hollow cylinder (11),
- a control module (80, 81, 82, 83, 90) attached to said inner body (10), said control module comprising an electronic board (90), a motor (81) and a motor wheel (82) caused to rotate by said motor (81),
- an outer body (20) receiving said inner body (10) and said control module,
- a thrust member (30) in contact with said tank (2) and mounted so as to be axially movable on said inner body (10) between a rest position and an actuation position,
- a control member (50) attached to said thrust member (30) and axially moving together therewith,
- a locking ring (40) mounted on said axial extension (15) of said inner body (10) so as to be capable of rotating between locking and releasing positions, said locking ring (40) being moved between the latter locking and releasing positions by said control module, said locking ring (40) co-operating in the locking position with said thrust member (30) so as to prevent axial movement of the latter and co-operating in the releasing position with said thrust member (30) so as to enable axial movement of the latter towards the actuation position thereof.

2. The device according to Claim 1, wherein said control module comprises a flexible element (83) attached on one side to said inner body (10) and on the other side to said locking ring (40), said motor wheel (82) co-operating with said flexible element (83) to deform it and thus move said locking ring (40) between its locking and releasing positions.

3. The device according to Claim 2, wherein said flexible element (83) is a wire, or a flexible blade.

4. The device according to any one of the preceding claims, wherein said axial extension (15) of said inner body (10) comprises a hollow axial cylinder (150) provided with at least one axial slot (151) extending over a portion of its height, said hollow axial cylinder (150) comprising at least one radial wall (155) extending radially outwards over the entire height of said hollow axial cylinder (150), said at least one radial wall (155) being angularly offset from each axial slot by 90°.

5. The device according to Claim 4, wherein said hollow axial cylinder (150) comprises two diametrically opposite slots and two diametrically opposed radial walls (155).

6. The device according to Claim 4 or 5, wherein said thrust member (30) comprises an axial well (32) extending axially downwards from an upper plate (33) co-operating with said tank (2), said axial well (32) comprising at least one radial extension (34) extending radially outwards from the outer surface of said axial well (32), the upper axial surface (340) of each radial extension (34) being arranged away from said upper plate (33), each radial extension (34) being arranged in a respective axial slot (151) of said inner body (10).

7. The device according to Claim 6, wherein said thrust member (30) comprises two radial extensions (34) which are diametrically opposed.

8. The device according to Claims 6 or 7, wherein said upper plate (33) of said thrust member (30) comprises, at an outer edge, a radial projection (31) for attaching a slider (70) co-operating during actuation with said control module.

9. The device according to any one of Claims 6 to 8, wherein said locking ring (40) comprises a hollow sleeve (41) provided with at least one first flat inner surface (44) extending radially inwards from an upper axial edge (411) of said hollow sleeve (41), each first flat inner surface (44) extending over a small part of the inner periphery of said hollow sleeve (41), said upper axial surface (340) of a radial extension (34) of said thrust member (30) being in axial abutment against a respective first flat inner surface (44) when the locking ring (40) is in the locking position.

10. The device according to Claim 9, wherein, in the releasing position of said locking ring (40), said at least one first flat inner surface (44) is angularly offset from said respective radial extension (34), thus allowing axial movement of said thrust member (30) relative to said inner body (10).

11. The device according to any one of Claims 6 to 10, wherein said at least one radial wall (155) is configured to co-operate with said locking ring (40) to define its locking and releasing positions.

12. The device according to any one of the preceding claims, wherein said locking ring (40) is configured to be blocked in the releasing position throughout the actuation cycle, and to return automatically to the locking position at the end of actuation.

13. The device according to Claims 2 and 12, wherein said control module is configured to deform said flexible element (83) from the start of the axial movement of the control member (50), so as to force said locking ring (40) to the locking position.

14. The device according to any one of the preceding claims, wherein said control module comprises time-out means to prevent, after each actuation of the dispenser (1), movement of said locking ring (40) from the locking position to the releasing position for a predetermined period of time.

15. The device according to Claim 14, wherein said time-out means are configured to block a control button of the device and/or said motor (81).

16. The device according to any one of the preceding claims, in which said fluid product is a pharmaceutical product, such as a drug.
